# FASCICULE DE BREVET EUROPEEN

(11) **EP 3 107 595 B1**
(45) Date de publication et mention de la délivrance du brevet: **09.12.2020**
(21) Numéro de dépôt: 15709266.9
(22) Date de dépôt: 17.02.2015
(51) Int. Cl.: A61L 31/10, A61L 31/14

(54) **DISPOSITIF IMPLANTABLE NOTAMMENT POUR LA RÉFECTION DE PAROI ABDOMINALE**
IMPLANTIERBARE VORRICHTUNG, INSBESONDERE ZUR REKONSTRUKTION DER BAUCHDECKE
IMPLANTABLE DEVICE ESPECIALLY FOR THE RECONSTRUCTION OF THE ABDOMINAL WALL

(30) Priorité: 19.02.2014 FR 1451316
(43) Date de publication de la demande: 28.12.2016
(73) Titulaire: Cousin Biotech, 59117 Wervicq Sud (FR)
(72) Inventeur: SOLECKI, Gilles, 59390 Lannoy (FR)
(74) Mandataire: Cabinet Beau de Loménie
(86) Numéro de dépôt international: PCT/FR2015/050383
(87) Numéro de publication internationale: WO 2015/124860

(56) Documents cités:
- WO-A1-2009/030867
- WO-A2-99/15210
- WO-A2-2005/058383
- US-A1- 2011 158 929
- DATABASE CA [Online] CHEMICAL ABSTRACTS SERVICE, COLUMBUS, OHIO, US; ABELEVICH, A. I. ET AL: "Endoprosthesis for paracolostomal hernias repair", XP002732338, extrait de STN Database accession no. 2014:49009 & RU 2 503 430 C1 (OOO REDPRIYATIE "REPER-NN", RUSSIA) 10 janvier 2014 (2014-01-10)
- DATABASE CA [Online] CHEMICAL ABSTRACTS SERVICE, COLUMBUS, OHIO, US; PARSHIKOV, V. V. ET AL: "Endoprosthesis for sutureless intra-abdominal plasty in ventral hernias", XP002732339, extrait de STN Database accession no. 2012:1320422 & RU 24604 94246 0494 C2 (OOO PREDPRIYATIE "REPER NN", RUSSIA) 10 septembre 2012 (2012-09-10)

## Description

La présente invention concerne le domaine technique des dispositifs implantables notamment destinés à réparer les défauts de la paroi abdominale, tels que les hernies inguinales et/ou les éventrations abdominales.

La présente invention concerne plus précisément des dispositifs implantables comprenant des implants textiles de renforcement dont l'une des faces est revêtue, au moins partiellement, d'un revêtement comprenant un polymère muco-adhésif en peropératoire.

Au sens de la présente invention, on entend par peropératoire, la période s'étendant du moment où le dispositif implantable est mis en contact avec la paroi à renforcer jusqu'à plusieurs heures après que l'intervention chirurgicale ne soit terminée, notamment environ cinq heures après la fin de ladite intervention.

### Arrière-plan de l'invention

On connait le document WO 2009/030867 décrivant un dispositif implantable comprenant un implant textile dont l'une des faces est revêtue au moins partiellement d'un revêtement comprenant un polymère de polyvinylpyrrolidone et 4% en poids de plastifiant par rapport au poids dudit polymère. Ledit plastifiant est par exemple le polyéthylèneglycol. Le polymère de polyvinylpyrrolidone n'est pas réticulé et est donc soluble au contact de l'eau contenue dans l'organisme. Ce revêtement est muco-adhésif par création de liaisons faibles de type Van Der Wall entre le revêtement et l'eau présents dans le mucus couvrant la paroi abdominale à renforcer. Ces liaisons faibles permettent un déplacement de la prothèse sans dégradation de l'implant textile mais celui-ci ne peut être réalisé par le chirurgien sans avoir une perte d'adhésion entre l'implant textile et la paroi abdominale à renforcer. Cette perte d'adhésion peut provoquer une migration de l'implant textile en peropératoire et ensuite une récidive de la hernie. Il est donc recommandé, mais difficile à faire pour un chirurgien inexpérimenté, de positionner l'implant textile en une seule fois sur la paroi abdominale à renforcer sans faire d'ajustement ultérieur, notamment en faisant glisser ledit implant textile dans un plan parallèle à ladite paroi abdominale.

Généralement, il est donc nécessaire, après avoir positionné l'implant textile sur la paroi musculaire à renforcer, d'ajuster son placement en la faisant glisser dans une direction parallèle au plan qui la comprend, ou en la décollant. Cet ajustement permet de centrer l'implant textile sur le défaut de la paroi.

Or, ce dispositif implantable ne peut pas subir un ajustement de sa position par un glissement ou par un décollement après son placement initial sans que la force d'adhésion entre l'implant textile et la paroi abdominale ne soit altérée.

Une explication non exhaustive à cette perte d'adhésion s'expliquerait par une migration d'une partie du revêtement auto-adhésif sur la paroi abdominale lors du déplacement ou du décollement de l'implant.

On connait également d'autres types de bioadhésifs composés de monomères qui polymérisent, éventuellement en se réticulant également, en peropératoires et permettent ainsi de fixer des implants textiles sur la paroi abdominale par l'intermédiaire de liaisons covalentes. Ces réactions de polymérisation en peropératoire, accompagnée éventuellement de réticulation, ont pour inconvénient d'être toujours exothermiques et ainsi d'engendrer des risques de nécrose des tissus. Ces adhésifs sont habituellement pulvérisés soit sur la paroi abdominale à renforcer, soit sur une face de l'implant textile lors de l'intervention chirurgicale. Il est donc difficile de contrôler la quantité d'adhésifs implantée. Ces adhésifs sont souvent des dérivés du sang ou des monomères de cyanoacrylates. Une fois la polymérisation terminée, le chirurgien n'a plus la possibilité de déplacer l'implant textile de la paroi abdominale par glissement sans rompre les liaisons covalentes et donc altérer les forces d'adhésion de l'implant textile sur la paroi. Il est donc nécessaire lorsqu'un implant textile est solidarisé à la paroi abdominale à renforcer par l'intermédiaire de liaisons covalentes formées entre le revêtement bioadhésif et ladite paroi que l'implant textile soit centré correctement dès son premier positionnement sur le défaut de la paroi sans nécessiter d'ajustements qui altèreraient la force d'adhésion de l'implant textile sur la paroi abdominale.

Le document WO 2012/064821 décrit un adhésif en partie composé de L-3-4-dihydroxyphenylalanine (DOPA) provenant des mollusques de type mytiloïde. Cet adhésif peut être disposé sous forme d'un revêtement sur l'une des faces d'un implant textile. L'adhésion entre l'implant textile et la paroi à renforcer est réalisée par des liaisons covalentes. De ce fait, l'implant textile n'est pas repositionnable en postopératoire sans une détérioration de la face de l'implant textile recevant le revêtement et/ou une altération de l'adhésion du dispositif implantable. En effet, les forces d'adhésion engagées entre l'implant textile revêtu du revêtement bioahésif et la paroi à renforcer sont trop importantes. L'exemple 35 du document WO 2012064821 montre une force de rupture d'environ 5.5+/-0.8 pounds (2.5 kg +/- 0.36 kg) pour désolidariser l'implant textile d'une membrane constituée de péricarde bovin. Cette désolidarisation dans la grande majorité des cas engendre une rupture de l'implant textile. Même si le chirurgien réussit à dissocier l'implant textile sans détériorer la surface textile de l'implant revêtue du revêtement bioadhésif, ni déchirer les tissus, cette dissociation se fait par rupture définitive des liaisons covalentes et donc par une perte complète des forces d'adhésion.

Le comportement d'un matériel étranger au contact de tissus vivants a été bien étudié auparavant par plusieurs auteurs. On assiste tout d'abord pour des prothèses ou implants textiles tricoté(e)s en polypropylène à une phase de réaction inflammatoire aiguë, exsudative puis cellulaire. Après la blessure tissulaire, par exemple la chirurgie abdominale, le processus de guérison normal débute. Il commence par une inflammation, caractérisée premièrement par une vasoconstriction et l'accumulation plaquettaire. La fibrine est ensuite formée pour colmater l'hémorragie et dure environ 15 minutes. Un phénomène parallèle est observé, par l'exsudation conséquente de protéines et des cellules plasma dans la zone affectée. La réponse cellulaire intervient entre 6 h et 16 h après le début de la lésion chirurgicale, quand une grande quantité de polymorphonucléaires neutrophiles apparaît, correspondant à la première vague de migration cellulaire. Ils restent de 3 à 5 jours, avec un sommet à 68 h. Déjà le 1er jour il y a une incursion de monocytes. Ceux-ci sont des précurseurs macrophages. La prolifération fibroblastique commence environ 36 h après la blessure chirurgicale. Les macrophages sont alors activés et les leucocytes prédominent dès le 3ème jour, quand ils atteignent un niveau maximal et persistent jusqu'à ce que la guérison soit complète. Cette première phase dure jusqu'au 2ème jour et peut durer jusqu'au 4ème jour postopératoire. Ces différentes cellules sont ensuite progressivement remplacées par des fibroblastes dont l'activité va s'intensifier avec production de collagène jusqu'à coloniser totalement la prothèse et l'intégrer en 4 à 6 semaines environ. Il existe donc une période critique avant que la réaction fibroblastique ne soit intense. C'est surtout pendant cette période que la stabilité de l'implant textile doit être assurée par des points de fixation. La faiblesse de la réaction inflammatoire est le reflet de la tolérance biologique alors que l'intensité de l'activité fibroblastique est le témoin d'une bonne résistance par la création d'un tissu cicatriciel de bonne qualité. De plus, le risque infectieux est proportionnel à la réaction inflammatoire locale. La prothèse idéale serait celle qui provoquerait une faible réaction inflammatoire et une intense activité fibroblastique. La durée de la réaction inflammatoire diffère selon les auteurs. Pour certains, elle disparaît en quelques semaines, pour d'autres, elle persiste plusieurs mois. Cette réaction dépend non seulement du matériau utilisé mais aussi de sa texture ou de sa porosité. Si la fixation primaire de l'implant textile est assurée par des sutures chirurgicales ou simplement par l'interposition de cet implant entre les plans de la paroi, il existe un risque de migration ou de mobilisation du renfort durant les jours qui suivent l'intervention. Il est classique de considérer que la fixation définitive (secondaire), obtenue par cicatrisation et intégration de l'implant textile dans la paroi, n'est acquise qu'après 1 mois à 6 semaines.

Il existe donc un besoin pour un dispositif implantable, notamment pour la réfection de paroi, comprenant un implant textile revêtu selon l'une de ses faces, et ce au moins partiellement d'un revêtement bioadhésif qui soit repositionnable sans perte d'adhésion, et ce de multiples fois.

Il existe également un besoin pour un dispositif implantable dont la quantité du revêtement bioadhésif puisse être contrôlée et reproductible, en particulier un revêtement bioadhésif qui puisse être stocké en même temps que ledit implant textile dans un sachet de stérilisation, et donc apte à subir une étape de stérilisation, par exemple à l'oxyde d'éthylène à l'état gazeux.

### Objet et résumé de l'invention

La présente invention a ainsi pour objet, selon un premier aspect, un dispositif implantable, notamment pour la réfection de paroi, selon les revendications 1 à 10. Ce dispositif comprend:
a) un implant textile de renforcement ayant des première et seconde faces, et
b) un revêtement bioadhésif revêtant au moins partiellement ladite première face, ledit revêtement comprenant au moins un polymère bioadhésif réticulé ionique choisi parmi les polymères suivants : un polymère d'acide acrylique, un polymère d'acide méthacrylique, un polymère d'acide itaconique, un polymère d'acide maléique ou un polymère d'anhydride maléique dont la fonction adhésive est activable en milieux aqueux.

Il a été trouvé que le polymère bioadhésif selon l'invention permet de solidariser l'implant textile selon sa face revêtue au moins partiellement dudit revêtement sur une paroi à renforcer, en particulier une paroi abdominale, sans perte d'adhésion dudit dispositif sur ladite paroi quand bien même ledit dispositif a été déplacé de ladite paroi par simple glissement, voir même décollé de ladite paroi.

Avantageusement, le polymère selon l'invention est réticulé de sorte qu'il n'est pas ou très peu hydrosoluble au contact de l'eau contenue dans l'organisme, ce qui évite une perte d'adhésion par dissolution d'une partie du revêtement.

En outre, le polymère selon l'invention adhère à la paroi à renforcer sans former de liaisons covalentes avec cette dernière, ni dégager de chaleur, de sorte qu'il est possible de déplacer par glissement ou de décoller le dispositif selon l'invention sans altérer la paroi abdominale ou encore ladite première face de l'implant textile.

Une explication non exhaustive est que le revêtement bioadhésif selon l'invention adhérerait par la formation de liaisons ioniques et/ou de liaisons de type Van Der Waals.

Le revêtement bioadhésif selon l'invention est stable, ce qui lui permet d'être disposé sur la première face de l'implant textile, subir une étape de stérilisation, ledit implant revêtu étant disposé dans un sachet de stérilisation. Avantageusement, le chirurgien n'a pas à pulvériser une colle sur l'implant textile juste avant l'intervention. En outre, la quantité et la disposition du revêtement selon la première face de l'implant textile peuvent être contrôlées lors du procédé de fabrication du dispositif, ce qui améliore la reproductibilité et la fiabilité de l'adhésion, ainsi que le confort du patient.

Au sens de la présente invention, on comprend par implant textile tout panneau tricoté, tressé ou tissé à partir d'éléments longilignes ainsi que tout panneau dans un ou plusieurs nontissé(s).

De préférence, l'implant textile est tricoté ou tissé, encore de préférence tricoté, plus particulièrement tricoté à mailles jetées.

Avantageusement, l'implant textile comprend des pores, débouchant selon la première face et/ou la seconde face, dont au moins une dimension est comprise entre 0,5 mm et 4 mm, de façon encore plus avantageuse entre 0,5 mm et 2 mm, en particulier de l'ordre du millimètre. Ces pores ou orifices peuvent traverser totalement ou non l'épaisseur de l'implant textile selon la présente invention. Plus avantageusement, ces pores traversent totalement l'implant textile, lequel a donc une structure ajourée.

Avantageusement, la seconde face dudit implant textile non revêtue favorise le développement de la fibrose cicatricielle et donc une fixation dudit implant par l'organisme.

Lesdits éléments longilignes peuvent être des fils monofilamentaires ou des fils multifilamentaires, de préférence des fils monofilamentaires.

Au sens de la présente invention, on entend par nontissé toute nappe de fibres orientées de façon aléatoire dans la direction longitudinale et/ou la direction transversale.

Dans un mode particulier de réalisation de la présente invention, l'implant textile selon la présente invention est en polymère biocompatible flexible et est non résorbable, c'est-à-dire qu'il reste de façon permanente dans le corps, ou semi-résorbable, c'est-à-dire que seulement une partie de l'implant textile reste de façon permanente dans le corps. L'implant textile semi-résorbable est fabriqué avec des fils en polymère résorbable et des fils en polymère non résorbable. Avantageusement, le polymère est un matériau synthétique ou semi-synthétique. De façon avantageuse, dans le cas où il est non résorbable, il est choisi parmi les polyéthylènes, polyesters, polypropylène, polytétrafluoroéthylène (PTFE), polyamide (6,6), polypropylène/PTFE, polyéthylène téréphtalate. En particulier, il s'agit du polypropylène. Dans le cas où l'implant textile est semi résorbable, les fils en polymère résorbable se trouvent sous la forme de polymère d'acide lactique de forme D ou L (PLLA ou PDLA) ou de copolymère d'acide lactique et glycolique (PLGA) tandis que les fils en polymère non résorbable sont choisis parmi les polyéthylènes, polyesters, polypropylène, polytétrafluoroéthylène (PTFE), polyamide (6,6), polypropylène/PTFE, polyéthylène téréphtalate. En particulier, par exemple, l'implant textile semi-résorbable est composé de fils résorbables en PLLA de diamètre 0,15 mm et de fils en polymère non résorbable en polypropylène de diamètre 0,1 mm.

Lorsque l'implant textile est destiné à la chirurgie par cœlioscopie, ses dimensions sont uniquement limitées par son utilisation chirurgicale, puisqu'il doit pouvoir, lorsqu'il est roulé, passer à l'intérieur d'un trocart ayant en général 1 cm ou 1,2 cm de diamètre. Par ailleurs, l'implant textile peut avoir toute géométrie nécessaire à son utilisation chirurgicale, qu'il soit à contour rectangulaire, carré, triangulaire, circulaire ou ovale, la seule condition étant qu'il puisse s'enrouler sur lui-même.

Avantageusement, l'implant textile selon la présente invention possède une résistance à la rupture supérieure à 16 N/cm pour les implants textiles destinés aux petites hernies inguinales, en particulier supérieure à 32 N/cm, pour les implants textiles destinés aux éventrations abdominales. Cette résistance est mesurée selon la norme ASTMD3787 suivant le test de Burst Strenght. Ce test consiste à utiliser une bille qui vient déformer l'implant textile tendu sur un anneau jusqu'à rupture. Le diamètre d'anneau est de 1 pouce (25,4 cm), le diamètre de la bille est de 0,38 inch (9,65 mm) pour une vitesse de test de 12 inchs/minutes (304,8 mm/minute). La résistance obtenue est divisée par le périmètre de l'anneau pour obtenir la résistance en N/cm.

Le revêtement bioadhésif peut être appliqué sur ladite première face de l'implant textile sous forme d'un film continu ou discontinu, sous forme de motifs uniformément répartis ou non, par exemple de bandes alternées ou de points.

Le revêtement bioadhésif peut être appliqué indifféremment selon la première ou la seconde face de l'implant textile.

Le revêtement adhésif peut être appliqué sur la première face de l'implant par enduction ou à la racle ou encore par immersion de la première face.

Au sens de la présente invention, on entend par revêtement bioadhésif, tout revêtement apte à coller aux tissus de l'organisme, en particulier à la paroi abdominale.

De préférence, l'implant textile a une masse surfacique inférieure ou égale à 50 g/m², avantageusement inférieure ou égale à 40 g/m², en particulier supérieure ou égale à 10 g/m², plus particulièrement supérieure ou égale à 20 g/m².

Avantageusement, le polymère bioadhésif selon l'invention est un homopolymère.

Le polymère bioadhésif selon l'invention est un polymère ionique, en particulier anionique. En effet, l'augmentation du pH provoque l'ionisation des groupes acides carboxyliques et corrélativement le gonflement dudit polymère sous forme de gel.

Le polymère bioadhésif selon l'invention peut ainsi former des complexes ioniques avec certains médicaments également ioniques, en particulier cationiques, en vue d'obtenir leur libération prolongée dans le temps.

La capacité d'adhésion améliorée du polymère bioadhésif selon l'invention combiné avec un implant textile, en particulier s'agissant de son caractère repositionnable sans perte d'adhérence sur la paroi abdominale, pourrait s'expliquer par le fait que ledit polymère est réticulé, mais également par sa capacité à former des liaisons ioniques avec la paroi abdominale.

Dans une variante, le polymère est réticulé par un agent de réticulation choisi parmi un polyéther polyalcénylique de sucre ou de polyalcool ou un divinyl glycol.

On entend par divinyl glycol, le 1,5-hexanediene-3,4-diol.

On entend, au sens de la présente invention, par polyalcool tout composé organique de formule CₙH₂ₙ₊₂Oₙ dans lequel n est compris entre 2 et 30, n étant un entier. La chaine alkyle dudit polyalcool étant saturée, linéaire ou ramifiée.

Dans une variante, l'agent de réticulation est choisi parmi un éther d'allyle de saccharose ou un allyle, diallyle, triallyle, ou tétraallyle de pentaérythritol.

On entend par allyle, tout groupe de formule -CH₂-CH=CH₂.

Dans une variante, le polymère bioadhésif est un carbomère.

Avantageusement, il s'agit d'un carbomère homopolymère, en particulier de type A selon le « Pharmacopeia Monograph Compendial Name » en vigueur aux USA après le 1er janvier 2006.

Dans une variante, le revêtement bioadhésif est obtenu à l'issue de la polymérisation d'une composition comprenant au moins un monomère choisi parmi : l'acide acrylique, l'acide méthacrylique, l'acide itaconique, l'acide maléique ou l'anhydride maléique, et un agent de réticulation, la proportion en poids dudit agent de réticulation par rapport au poids total en monomère(s) est supérieur à 0% et inférieure ou égale à 4%, plus particulièrement inférieure ou égale à 3%, encore plus particulièrement inférieure ou égale à 2%, avantageusement supérieure ou égale à 0,10%, plus avantageusement supérieure ou égale à 1%.

La quantité d'agent de réticulation est très faible, ce qui permet d'obtenir un polymère bioadhésif légèrement réticulé mais insoluble dans l'eau.

De préférence, ladite composition comprend également un solvant, par exemple l'acétate d'éthyle.

Le polymère est déshydraté et apte à former un gel en milieu aqueux.

Le polymère n'est pas sous la forme d'un gel avant implantation. Il est donc déshydraté. Au sens de la présente invention, on entend par « polymère déshydraté » un polymère contenant moins de 1% en poids d'eau par rapport au poids total du polymère. Après implantation, le milieu d'implantation étant un milieu aqueux, le polymère absorbe l'eau du milieu aqueux et se transforme en gel. Avantageusement, ce polymère ne forme pas de gel pour un taux d'humidité relative ambiante inférieure ou égale à 65% d'humidité relative (HR). Avantageusement, le polymère absorbe au moins 20% en poids d'eau par rapport au poids total du polymère pour former un gel lors de son implantation.

Au sens de la présente invention, on entend par « gel » tout matériau constitué par un réseau polymérique retenant un liquide. Les gels possèdent une propriété commune avec les solides, celle de ne pas s'écouler sous son propre poids mais aussi une caractéristique des liquides, celle de se déformer sous l'effet d'une certaine contrainte. En quelque sorte, on peut assimiler « l'état gel » comme l'intermédiaire entre l'état liquide et l'état solide. Dans le cadre de la présente invention, le liquide retenu par le réseau polymère est une solution aqueuse, en particulier de l'eau.

Avantageusement, le polymère déshydraté est apte à former un gel en milieu aqueux à la température corporelle, c'est-à-dire d'environ 36 - 37°C. Ainsi, dans ce mode de réalisation particulier, le polymère ne forme pas de gel à une température inférieure à 35°C, avantageusement inférieure ou égale à 30°C et en particulier à une température proche de la température ambiante, c'est-à-dire à 20 - 25°C.

De façon avantageuse, le polymère déshydraté apte à former un gel en milieu aqueux est tel que le gel obtenu a une teneur en eau d'au moins 20% en poids lorsqu'il est totalement hydraté à 37°C, de manière avantageuse d'au moins 30% en poids, en particulier comprise entre 75 et 99% en poids.

Dans une mode de réalisation particulier, le polymère déshydraté forme un gel au plus tard dans les 15 minutes de sa mise en contact avec un milieu aqueux, c'est-à-dire dans le cas de la présente invention au plus tard dans les 15 minutes de son implantation.

Dans une variante, la viscosité Brookfield du polymère bioadhésif à 25°C, dispersé à 0,5% en poids dans de l'eau déminéralisée, à un pH compris entre 6 et 8, est supérieure à 4 Pa.s (4 000 cPoises) de préférence inférieure à 11 Pa.s (11 000 cPoises).

Plus particulièrement, la viscosité Brookfield RVT, 20 rpm, peut être mesurée sur un polymère neutralisé à un pH compris entre 7,3 et 7,8, à 0,5% en poids avec une pâle n°5 selon la norme Lubrizol 430-I.

Dans une variante, le polymère bioadhésif a une teneur en acide carboxylique supérieure ou égale à 40%, de préférence supérieure ou égale à 50%.

Avantageusement, la teneur en acide carboxylique est mesurée selon la norme Lubrizol 1318-A.

Plus particulièrement, la teneur en acide carboxylique est inférieure ou égale à 75%, encore plus particulièrement inférieure ou égale à 70%.

Dans une variante, la masse surfacique (g/m²) du revêtement est supérieure ou égale à la masse surfacique de l'implant textile (g/m²).

De préférence, la masse surfacique du revêtement est supérieure ou égale à une fois et demie la masse surfacique de l'implant textile.

Dans une variante, le revêtement comprend un plastifiant, de préférence choisi parmi les polyalcools, de préférence le polyéthylene glycol.

On entend, au sens de la présente invention, par polyalcool tout composé organique de formule CₙH₂ₙ₊₂Oₙ dans lequel n est compris entre 2 et 30, n étant un entier. La chaine alkyle dudit polyalcool étant saturée, linéaire ou ramifiée.

On entend par « plastifiant » au sens de la présente invention, toute substance, autre que les molécules d'eau, apte à diminuer la température de transition vitreuse du polymère selon l'invention.

Le plastifiant selon l'invention facilite la pose du revêtement bioadhésif selon la première face de l'implant textile.

Avantageusement, le plastifiant a une masse moléculaire moyenne en poids Mw comprise entre 100g/mol et 700 g/mol.

Dans une variante, l'implant textile comprend au moins un fil monofilamentaire, notamment en polypropylène, ayant un diamètre supérieur ou égal à 0,10 mm et inférieur ou égal à 0,60 mm, de préférence inférieur ou égal à 0,30 mm.

La présente invention a pour objet, selon un second aspect, un dispositif comprenant un sachet de stérilisation comprenant le dispositif implantable selon l'une quelconque des variantes de réalisation précédentes, de préférence l'implant textile est dans une configuration plane.

Avantageusement, le revêtement bioadhésif selon l'invention peut être disposé sur la première face de l'implant textile dans un sachet de stérilisation et ainsi stocké jusqu'à son utilisation.

La stérilisation peut être effectuée en soumettant le sachet de stérilisation comprenant le dispositif implantable selon l'invention à des rayons gamma ou à de l'oxyde d'éthylène à l'état gazeux.

Avantageusement, ledit sachet est perméable à l'oxyde d'éthylène à l'état gazeux.

### I - Description d'un premier protocole permettant d'évaluer la force d'adhésion d'un implant textile après une mise en position et un ajustement en peropératoire comprenant les étapes suivantes :

- Etape 1 : L'animal est placé sur le champ opératoire stérile dans des conditions de température T = 23°C et d'humidité relative HR = 35%. L'animal est ensuite anesthésié par injection de drogue constituée de Ketamine (10 mg/kg), d'Azaperone (2 mg/kg) et d'Atropine (0.05 mg/kg).
- Etape 2 : Une incision abdominale médiane est réalisée au bistouri sur une longueur de 20 cm et deux autres incisions de longueur 20 cm sont réalisées perpendiculairement aux extrémités de cette incision. Ces incisions permettent un décollement de la peau et de la couche graisseuse afin d'accéder ensuite à la paroi abdominale de l'animal.
- Etape 3 : L'implant textile mis en place est carré de dimension 7 cm x 7 cm. Une première extrémité d'un fil en polyester USP 3-0 est nouée au centre de l'implant textile, l'autre extrémité du fil comporte une boucle de diamètre 30 mm qui sera solidarisée à un dynanomètre après l'ajustement de l'implant textile. L'implant textile est mis en position sur la paroi abdominale droite de l'animal et reste en place pendant une durée de 5 secondes puis subit un décollement complet. L'implant textile est ensuite mis à nouveau en position au même endroit sur la paroi abdominale pendant une durée de 5 secondes. Il subit ensuite un glissement par traction manuelle selon son plan sur une distance de 2 cm dans le sens perpendiculaire à l'axe de l'animal. Après une durée de 2 mn, on mesure de façon dynamométrique la force d'adhésion (F_{A}) de l'implant textile sur la paroi abdominale par traction sur le fil polyester. Le dynamomètre numérique de marque CHATILLON® modèle DFE II est utilisé pour réaliser cette mesure.
- L'animal est ensuite euthanasié.

### II - Description d'un second protocole permettant d'évaluer la force d'adhésion de l'implant textile après une mise en position, un ajustement et une durée d'implantation déterminée comprenant les étapes suivantes :

- Les étapes 1 et 2 du premier protocole sont reproduites.
- La prothèse mise en place est carrée de dimension 7 cm x 7 cm. Une première extrémité d'un fil en polyester USP 3-0 est nouée au centre de la prothèse, l'autre extrémité du fil comporte une boucle de diamètre 30 mm qui permettra après une durée d'implantation une fixation sur un dynamomètre. La prothèse est mise en position sur la paroi abdominale droite de l'animal et reste en place pendant une durée de 5 secondes puis subit un décollement complet. La prothèse est ensuite mise à nouveau en position au même endroit sur la paroi abdominale pendant une durée de 5 secondes. Elle subit ensuite un glissement par traction manuelle selon son plan sur une distance de 2 cm dans le sens perpendiculaire à l'axe de l'animal. C'est à partir de cet instant que l'on commence la mesure de la durée d'implantation à l'aide d'un chronomètre.
- Les incisions sont ensuite refermées par suture au fil et l'animal est ensuite réveillé.
- Après une période déterminée l'animal est à nouveau anesthésié au bloc opératoire. Après retrait des fils de suture et ouverture des incisions, la prothèse est attachée à un dynamomètre par le fil USP 3-0 en polyester pour une mesure de sa force d'adhésion. C'est à cet instant que la durée d'implantation est mesurée et que la force d'adhésion (F_{B}) est enregistrée avec un dynamomètre de marque CHATILLON® modèle DFE II.
- L'animal est ensuite euthanasié.

### III - Description d'un exemple de réalisation d'un dispositif implantable (C) selon l'invention comparativement à un implant textile (A) non revêtu et à un dispositif implantable comprenant un revêtement bioadhésif à base de PVP (B) dont les forces d'adhésion (F_{A}) et (F_{B}) ont été évaluées selon les premier et second protocoles décrits respectivement aux points I et II

### 1- Implant textile (A) non revêtu d'un revêtement bioadhésif

L'implant textile (A) est un panneau tricoté à mailles jetées réalisé avec deux fils monofilamentaires de polypropylène de diamètre 0.10 mm sur un métier Rachel possédant 28 aiguilles au pouce (1 pouce = 25.4 mm). La structure de base est réalisée à l'aide de deux barres à passettes avec un enfilage un plein - un vide sur chaque barre. Chaque barre à passettes supporte un fil monofilamentaire.

L'armure de tricotage est la suivante :
mouvement première barre : 4-2 / 2-0 / 2-4 / 4-6 //
mouvement seconde barre : 4-6 / 6-8 / 6-4 / 4-2 //

### 2- Dispositif implantable (B)

L'implant textile est un panneau tricoté à mailles jetées revêtu d'un revêtement bioadhésif. Le panneau tricoté est réalisé avec deux fils monofilamentaires de polypropylène de diamètre 0.10 mm sur un métier Rachel possédant 28 aiguilles au pouce (1 pouce = 25.4 mm). La structure de base est réalisée à l'aide de deux barres à passettes avec un enfilage un plein - un vide sur chaque barre. Chaque barre à passettes supportant un fil monofilamentaire.

L'armure de tricotage est la suivante :
mouvement première barre : 4-2 / 2-0 / 2-4 / 4-6 //
mouvement seconde barre : 4-6 / 6-8 / 6-4 / 4-2 //

Le revêtement bioadhésif est composé de polyvinylpyrrolidone (PVP) commercialisé par BASF® sous le nom de Kollidon 90® et de polyéthylèneglycol (PEG) commercialisé par BASF® sous le nom de Lutrol E400®.

Les proportions des différents composants dans le dispositif implantable (B) sont les suivantes :
- Masse surfacique totale du dispositif implantable (B) : 194.8 g/m²
- Masse surfacique de l'implant textile non revêtu : 35 g/m²
- Masse surfacique du revêtement bioadhésif : 159.8 g/m2
- Masse surfacique de Kollidon 90 ®: 158 g/m²
- Masse surfacique de Lutrol E400 : 1.80 g/m2

### 3- Dispositif implantable (C) selon l'invention

L'implant textile est un panneau tricoté à mailles jetées revêtu d'un revêtement bioadhésif. Le panneau tricoté est réalisé avec deux fils monofilamentaires de polypropylène de diamètre 0.10 mm sur un métier Rachel possédant 28 aiguilles au pouce (1 pouce = 25.4 mm). La structure de base est réalisée à l'aide de deux barres à passettes avec un enfilage un plein - un vide sur chaque barre. Chaque barre à passettes supporte un fil monofilamentaire.

L'armure de tricotage est la suivante :
mouvement première barre : 4-2 / 2-0 / 2-4 / 4-6 //
mouvement seconde barre : 4-6 / 6-8 / 6-4 / 4-2 //

Le revêtement bioadhésif est composé d'un acide polyacrylique réticulé par l'allyl de pentaerythritol commercialisé par la société Noveon® et de Polyéthylèneglycol (PEG) en tant que plastifiant commercialisé par BASF® sous le nom de Lutrol E400®.

Les proportions des différents composés dans le dispositif implantable (C) sont les suivantes :
- Masse surfacique totale du dispositif implantable : 86.5 g/m²
- Masse surfacique de l'implant textile : 30 g/m²
- Masse surfacique du revêtement bioadhésif : 56.5 g/m²
- Masse surfacique de l'acide polyacrylique réticulé par l'allyl de pentaérythritol : 48.2 g/m²
- Masse surfacique de Lutrol E400® : 8.3 g/m²

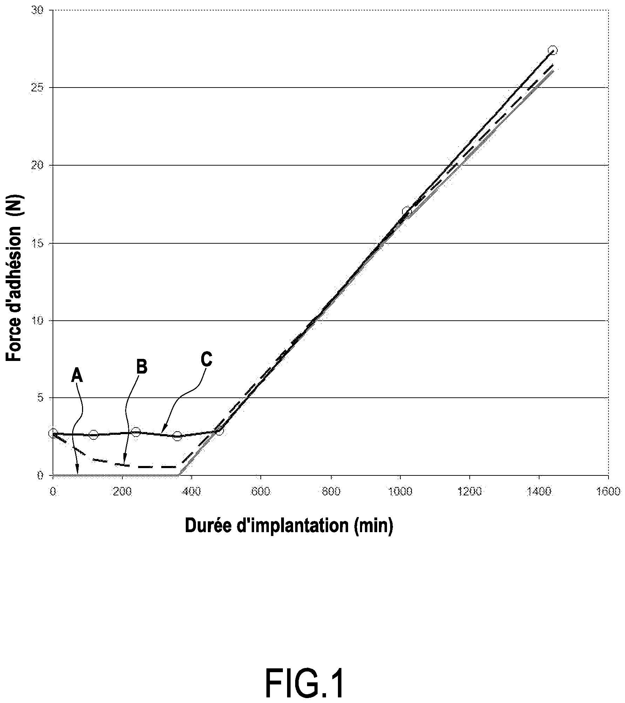

### IV - Description de la figure 1

La figure 1, annexée à la présente, représente les trois courbes des forces d'adhésion F_{A} et F_{B} mesurées pour l'implant textile (A) et les dispositifs implantables (B) et (C) selon les protocoles I et II effectués sur un modèle porcin. Les premiers points mesurés au terme de 2 mn correspondent à la force d'adhésion F_{A}, les points mesurés après 2 mn d'implantation, correspondent aux forces d'adhésion F_{B}.

### V - Résultats d'essais

D'après les trois courbes représentées à la figure 1, on observe que pour une durée d'implantation inférieure à 8h00 (480 mn), les forces d'adhésion de l'implant (A) et des dispositifs (B) et (C) sont inférieures à 3 Newtons. Pour une durée d'implantation supérieure à 480 mn, la cicatrisation apparaît pour laisser place à une colonisation cellulaire de la prothèse, d'où la très nette élévation des forces d'adhésion obtenue. Du fait de l'apparition de la réaction inflammatoire aiguë, exsudative puis cellulaire, l'adhésion de l'implant textile (A) et des dispositifs (B) et (C) aux tissus augmente considérablement et de façon linéaire à partir de ce moment.

Avantageusement, on observe que la force d'adhésion du dispositif implantable (C) est stable et comprise dans l'intervalle [2.5 ; 2.9] Newtons pour une durée d'implantation inférieure à 480 mn et ce malgré un ajustement en peropératoire. Au-delà de 480 mn, l'adhésion est assurée par les phénomènes de colonisation cellulaire de l'implant textile (A) ou des dispositifs implantables (B) et (C) et augmente ainsi linéairement de façon importante.

Le tableau 1 ci-dessous reprend les valeurs F_{A} et F_{B} obtenues pour l'implant textile (A) et les dispositifs implantables (B) et (C) issues de la figure 1.

**Tableau 1**

| Durée d'implantation (en minutes) | Implant textile (A) | Dispositif implantable (B) | Dispositif implantable (C) |
|---|---|---|---|
| 2 | 0.0 N | 2.6 N | 2.7 N |
| 120 | 0.0 N | 1.0 N | 2.6 N |
| 240 | 0.0 N | 0.5 N | 2.8 N |
| 360 | 0.0 N | 0.5 N | 2.5 N |
| 480 | 3.0 N | 3.3 N | 2.9 N |
| 1020 | 16.6 N | 16.8 N | 17.0N |
| 1440 | 26.0 N | 26.5 N | 27.4 N |

## Revendications

1. Dispositif implantable, notamment pour la réfection de paroi, **caractérisé en ce qu'**il comprend:
- un implant textile de renforcement ayant des première et seconde faces,
- un revêtement bioadhésif revêtant au moins partiellement ladite première face, ledit revêtement comprenant au moins un polymère bioadhésif réticulé ionique apte à solidariser l'implant textile selon sa première face revêtue au moins partiellement dudit revêtement bioadhésif sur une paroi à renforcer, ledit au moins un polymère bioadhésif étant choisi parmi les polymères suivants : un polymère d'acide acrylique, un polymère d'acide méthacrylique, un polymère d'acide itaconique, un polymère d'acide maléique ou un polymère d'anhydride maléique, dont la fonction adhésive est activable en milieux aqueux, **en ce que** ledit revêtement est apte à coller aux tissus de l'organisme, et **en ce que** ledit au moins un polymère est déshydraté et apte à former un gel en milieu aqueux.

2. Dispositif implantable selon la revendication 1, **caractérisé en ce que** le polymère est réticulé par un agent de réticulation choisi parmi un polyéther polyalcénylique de sucre ou de polyalcool ou un divinyl glycol.

3. Dispositif implantable selon la revendication 2, **caractérisé en ce que** l'agent de réticulation est choisi parmi un éther d'allyle de saccharose ou un allyle, diallyle, triallyle, ou tétraallyle de pentaérythritol.

4. Dispositif implantable selon l'une quelconque des revendications 1 à 3, **caractérisé en ce que** le polymère bioadhésif est un carbomère.

5. Dispositif implantable selon l'une quelconque des revendications 1 à 4, **caractérisé en ce que** le revêtement bioadhésif est obtenu à l'issue de la polymérisation d'une composition comprenant au moins un monomère choisi parmi : l'acide acrylique, l'acide méthacrylique, l'acide itaconique, l'acide maléique ou l'anhydride maléique, et un agent de réticulation, la proportion en poids dudit agent de réticulation par rapport au poids total en monomère(s) est supérieur à 0% et inférieure ou égale à 4%.

6. Dispositif implantable selon l'une quelconque des revendications 1 à 5, **caractérisé en ce que** la viscosité Brookfield du polymère bioadhésif à 25°C, dispersé à 0,5% en poids dans de l'eau déminéralisée, à un pH compris entre 6 et 8, est supérieure à 4 Pa.s (4 000 cPoises) de préférence inférieure à 11 Pa.s (11 000 cPoises).

7. Dispositif implantable selon l'une quelconque des revendications 1 à 6, **caractérisé en ce que** la masse surfacique (g/m²) du revêtement est supérieure ou égale à la masse surfacique de l'implant textile (g/m²).

8. Dispositif implantable selon l'une quelconque des revendications 1 à 7, **caractérisé en ce que** le revêtement comprend un plastifiant, de préférence choisi parmi les polyalcools.

9. Dispositif implantable selon l'une quelconque des revendications 1 à 8, **caractérisé en ce que** l'implant textile comprend au moins un fil monofilamentaire, notamment en polypropylène, ayant un diamètre supérieur ou égal à 0,10 mm et inférieur ou égal à 0,60 mm.

10. Dispositif implantable selon l'une quelconque des revendications 1 à 9, **caractérisé en ce que** le polymère bioadhésif a une teneur en acide carboxylique supérieure ou égale à 40%.

11. Dispositif caractérisé en en ce qu'il comprend un sachet de stérilisation comprenant le dispositif implantable selon l'une quelconque des revendications 1 à 10, de préférence l'implant textile est dans une configuration plane.

## Patentansprüche

1. Implantierbare Vorrichtung, insbesondere zur Wiederherstellung einer Wand, **dadurch gekennzeichnet, dass** sie umfasst:
- ein textiles Implantat zur Verstärkung, das eine erste und eine zweite Seite aufweist,
- eine Bioklebstoffbeschichtung, die zumindest zum Teil die erste Seite beschichtet, wobei die Beschichtung zumindest einen vernetzten ionischen Polymer-Bioklebstoff umfasst, der dazu geeignet ist, das textile Implantat an seiner ersten Seite, die zumindest zum Teil mit der Bioklebstoff-Beschichtung beschichtet ist, auf einer zu verstärkenden Wand zu befestigen, wobei der zumindest eine Polymer-Bioklebstoff aus den folgenden Polymeren ausgewählt ist: einem Acrylsäure-Polymer, einem Methacrylsäure-Polymer, einem Itaconsäure-Polymer, einem Maleinsäure-Polymer oder einem Malein(säure)anhydrid-Polymer, dessen klebende Funktion in wässrigem Medium aktivierbar ist, und dass die Beschichtung geeignet ist, an Geweben des Organismus zu kleben, und dass das zumindest eine Polymer dehydriert und dazu geeignet ist, in wässrigem Medium ein Gel zu bilden.

2. Implantierbare Vorrichtung nach Anspruch 1, **dadurch gekennzeichnet, dass** das Polymer durch ein Vernetzungsmittel vernetzt ist, das aus einem Polyalkenyl-Polyether von Zucker oder von einem Polyalkohol oder einem Divinylglycol ausgewählt ist.

3. Implantierbare Vorrichtung nach Anspruch 2, **dadurch gekennzeichnet, dass** das Vernetzungsmittel aus einem Allylether von Saccharose oder einem Pentaerythritol-Allyl, -Diallyl, -Triallyl oder -Tetraallyl ausgewählt ist.

4. Implantierbare Vorrichtung nach einem der Ansprüche 1 bis 3, **dadurch gekennzeichnet, dass** der Polymer-Bioklebstoff ein Carbomer ist.

5. Implantierbare Vorrichtung nach einem der Ansprüche 1 bis 4, **dadurch gekennzeichnet, dass** die Bioklebstoff-Beschichtung am Ende der Polymerisation einer Zusammensetzung erhalten wird, die zumindest ein Monomer umfasst, das ausgewählt ist aus: Acrylsäure, Methacrylsäure, Itaconsäure, Maleinsäure oder Malein(säure)anhydrid, sowie ein Vernetzungsmittel, wobei der Gewichtsanteil des Vernetzungsmittels bezogen auf das Gesamtgewicht des Monomers bzw. der Monomere höher als 0 % und kleiner oder gleich 4 % ist.

6. Implantierbare Vorrichtung nach einem der Ansprüche 1 bis 5, **dadurch gekennzeichnet, dass** die Viskosität nach Brookfield des Polymer-Bioklebstoffs bei 25 °C, dispergiert auf 0,5 Gew.-% in entmineralisiertem Wasser, bei einem pH zwischen 6 und 8, höher als 4 Pa.s (4.000 cPoises), vorzugsweise niedriger als 11 Pa.s (11.000 cPoises) ist.

7. Implantierbare Vorrichtung nach einem der Ansprüche 1 bis 6, **dadurch gekennzeichnet, dass** das Flächengewicht (g/m²) der Beschichtung höher als das oder gleich dem Flächengewicht des textilen Implantats (g/m²) ist.

8. Implantierbare Vorrichtung nach einem der Ansprüche 1 bis 7, **dadurch gekennzeichnet, dass** die Beschichtung einen Weichmacher umfasst, der vorzugsweise aus den Polyalkoholen ausgewählt ist.

9. Implantierbare Vorrichtung nach einem der Ansprüche 1 bis 8, **dadurch gekennzeichnet, dass** das textile Implantat zumindest einen Monofil-Faden umfasst, insbesondere aus Polypropylen, mit einem Durchmesser von mehr als oder gleich 0,10 mm und kleiner als oder gleich 0,60 mm.

10. Implantierbare Vorrichtung nach einem der Ansprüche 1 bis 9, **dadurch gekennzeichnet, dass** der Polymer-Bioklebstoff einen Gehalt an Carbonsäure von mehr als oder gleich 40 % aufweist.

11. Vorrichtung, die **dadurch gekennzeichnet ist, dass** sie einen Sterilisationsbeutel umfasst, der die implantierbare Vorrichtung nach einem der Ansprüche 1 bis 10 umfasst, wobei das textile Implantat vorzugsweise in einer flachen Ausgestaltung vorliegt.

## Claims

1. An implantable device, in particular for wall repair, **characterized in that** it comprises:
- a reinforcing textile implant having first and second surfaces;
- a bioadhesive coating to coat said first surface at least in part, said coating comprising at least one ionic, cross-linked bioadhesive polymer able to bond the textile implant on its first surface coated at least in part with said bioadhesive coating onto a wall to be reinforced, said at least one bioadhesive polymer being selected from among the following polymers: an acrylic acid polymer, a methacrylic acid polymer, an itaconic acid polymer, a maleic acid polymer or a maleic anhydride polymer having an adhesive function that can be activated in an aqueous medium, **in that** said coating is able to adhere to body tissues, and **in that** said at least one polymer is dehydrated and capable of forming a gel in an aqueous medium.

2. The implantable device according to claim 1, **characterized in that** the polymer is cross-linked by a cross-linking agent selected from among a sugar polyalkenyl polyether or polyalcohol polyalkenyl polyether or a divinyl glycol.

3. The implantable device according to claim 2, **characterized in that** the cross-linking agent is selected from among a sucrose allyl ether or allyl, diallyl, triallyl or tetraallyl pentaerythritol.

4. The implantable device according to any one of claims 1 to 3, **characterized in that** the bioadhesive polymer is a carbomer.

5. The implantable device according to any one of claims 1 to 4, **characterized in that** the bioadhesive coating is obtained after the polymerisation of a composition comprising at least one monomer selected from among: acrylic acid, methacrylic acid, itaconic acid, maleic acid or maleic anhydride, and a cross-linking agent, the weight proportion of said cross-linking agent relative to the total weight of monomer(s) is higher than 0% and lower than or equal to 4%.

6. The implantable device according to any one of claims 1 to 5, **characterized in that** the Brookfield viscosity of the bioadhesive polymer at 25°C, dispersed at 0.5 in weight % in demineralised water, at a pH of between 6 and 8, is higher than 4 Pa.s (4,000 cPoises), preferably lower than 11 Pa.s (11,000 cPoises).

7. The implantable device according to any one of claims 1 to 6, **characterized in that** the mass per unit area (g/m²) of the coating is higher than or equal to the mass per unit area of the textile implant (g/m²).

8. The implantable device according to any one of claims 1 to 7, **characterized in that** the coating comprises a plasticizer, preferably selected from among polyalcohols.

9. The implantable device according to any one of claims 1 to 8, **characterized in that** the textile implant comprises at least one monofilament yarn, in polypropylene in particular, having a diameter greater than or equal to 0.10 mm and smaller than or equal to 0.60 mm.

10. The implantable device according to any one of claims 1 to 9, **characterized in that** the bioadhesive polymer has a carboxylic acid content higher than or equal to 40%.

11. A device **characterized in that** it comprises a sterilisation pouch comprising the implantable device according to any one of claims 1 to 10, preferably the textile implant being in a planar configuration.
